Europäisches Patentamt

European Patent Office

Office européen des brevets

(11) Publication number: **0 446 509 A1**

# EUROPEAN PATENT APPLICATION

(21) Application number: **90302772.0**

(22) Date of filing: **15.03.90**

(51) Int. Cl.5: **A61K 35/52**, A01K 67/02

(43) Date of publication of application:
**18.09.91 Bulletin 91/38**

(84) Designated Contracting States:
**DE FR GB**

(71) Applicant: **Zavos, Panayiotis M.**
**2413 Vince Road**
**Nicholasville, Kentucky 40356(US)**

(72) Inventor: **Zavos, Panayiotis M.**
**2413 Vince Road**
**Nicholasville, Kentucky 40356(US)**

(74) Representative: **Perry, Robert Edward et al**
**GILL JENNINGS & EVERY 53-64 Chancery Lane**
**London WC2A 1HN(GB)**

(54) Semen filter column.

(57) A semen filter device comprising a column having openings at both ends, dehydrated particulate filtering means in said column; said filtering means capable of being hydrated to form a multi-layer filter, and retaining means in said column to retain said dehydrated filtering means in said column.

Field of the Invention

This invention relates to a device and method for processing semen and filtering it through a filtration column for the purpose of selectively increasing the number of motile and morphologically normal spermatozoa in the filtrate.

Background of the Invention

Several methods have been proposed, whereby semen can be processed to increase the quality and quantity of spermatozoa population in a semen sample, thereby increasing the probability of success when the semen sample is used for Artificial Insemination (AI), In Vitro Fertilization (IVF), and related clinical techniques. Among these methods are washing and storing spermatozoa in media, and the "rise" or "swim up" procedure, a technique that takes advantage of the swimming abilities of a small percentage of spermatozoa within a population. See e.g. Russell and Rogers, J. Androl., 8:25-33, 1987.

None of these methods are simple and short and all of them are susceptible to technician error and accident. However, these disadvantages are overcome by the present invention, which provides for a simple apparatus and procedure that enhances normal morphologic spermatozoa forms and increases the number of motile spermatozoa. Semen that is processed and filtered in accordance with this invention demonstrates qualitative and quantitative spermatozoa characteristics that are as good or better than any other semen processing method. Data from tests made on the procedure and apparatus claimed in this invention are set forth in Table 1, which is attached hereto.

Summary of the Invention

The present invention is a sterile, non-hydrated, disposable filtration column to be manufactured in accordance with strict quality controls, packaged in sterile wrapping, and made available for wide distribution to clinical locations. It consists of a disposable column, a top stopper, a removable, nonporous upper disc situated horizontally across the column approximately two-thirds from the top, a bottom filter disc made of standard insulate material that allows for the free flow of elements less than 50 to 100 microns in size, numerous dehydrated sephadex beads, 40 to 120 microns in diameter, that lay on top of the insulate disc, and a reuseable bottom closure cap. The device is used to filter spermatozoa after it has been prepared for filtration in accordance with a semen processing method that is part of the invention.

By virtue of the present invention, it is possible to process semen and filter spermatozoa for the purpose of selectively increasing the normal morphology, motility, sperm concentration and fertilization potential of semen samples for use in AI and related clinical techniques. Further, it is possible to filter spermatozoa easily and quickly, with good repeatability and with little likelihood of technician error or accident. The invention employs a sterile, non-hydrated, disposable filtration column that can be manufactured and distributed widely to clinical locations, where it can be hydrated and used with relative ease.

Detailed Description of the Invention

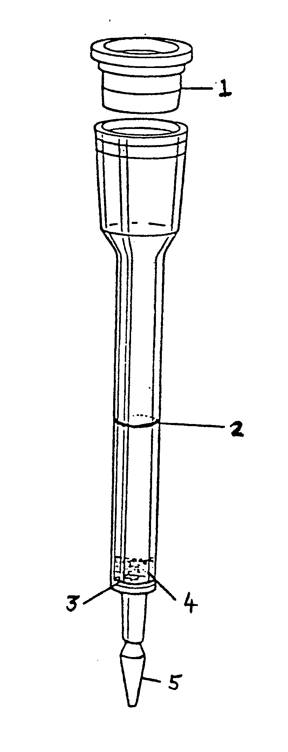

The invention will now be described by way of example only with reference to the accompanying lateral perspective view cf a device embodying the present invention. The drawing shows a disposable semen filter column having a top stopper (1), a nonporous upper disc (2) located horizontally across the column approximately two-thirds of the column height from the top, a lower disc (3) made of standard insulate material that allows for the free flow of elements less than 50 to 100 microns in size, numerous dehydrated sephadex beads (4), 40 to 120 microns in diameter, that lie on top of the insulate disc, and a reuseable bottom closure cap (5).

Use of the present invention involves two major steps:

(A) Mixing and washing the semen in order to prepare the spermatozoa for filtration; and
(B) Filtering the spermatozoa through the Semen Filter Column.

The spermatozoa are prepared for filtration by obtaining a freshly ejaculated and liquified semen specimen and evaluating it for volume, sperm count per milliliter, motility and presence of debris. These findings should be noted for later reference. Next, the semen specimen is diluted in a medium. Any standard laboratory medium accepted in the literature for use with human semen is acceptable. The liquid should be mixed gently and centrifuged at 400g for 6 minutes. The supernatant is then removed and discarded. The resulting sperm pellet is resuspended in media to a final concentration of $100 \times 10^6$ sperm/milliliter. With the mixing and washing of the semen complete, the spermatozoa are now ready for filtration.

Prior to or at the beginning of semen preparation, a Semen Filter Column (SFC) should be removed from its sterile package. One or more SFCs may be needed per ejaculate to be processed. At this same time, a 15 ml. test tube should be placed upright in a 37 degree centigrade water bath.

Holding the SFC upright and with the bottom closure cap in place, the top stopper should be removed and 3.0 ml. of media placed into the SFC. The media should be at room temperature. The media will flow onto the nonporous upper disc, which should then be gently removed by nudging it with a pipette so as to push the disc against the walls of the SFC, and pulling it upward. The medium can now drip down the column and mix with the dehydrated sephadex beads on the lower insulate disc. A Pasteur pipette should be used to mix the beads with the medium and remove any air bubbles that may exist or have formed in the bottom of the SFC. Caution should be taken not to disturb the bottom disc.

Once the medium has begun mixing with the dry beads, five minutes or more should be allowed for the beads to expand and settle thereby forming a multi-layer filter on the insulate disc. Next, remove the bottom cap from the SFC in order to allow two to three drops of media to drip through the bottom opening. The opening is quickly closed with the bottom closure cap and the SFC is placed into the 37 degree centigrade water bath until the semen is ready for filtration.

When ready to start the actual filtration, remove the SFC from the water bath. If the initial evaluation of the semen showed little or no debris and motility greater than 50%, place 0.75 ml. of the well mixed-washed semen preparation into the top of the SFC. If, however, debris is excessive and motility is less than 50%, only 0.5 ml. of the semen preparation should be placed into the SFC.

Once the semen preparation is in the filtration column, the bottom closure cap should be removed and the SFC placed into the 15 ml. test tube so that as the filtrate flows through the SFC it can be collected in the test tube. The semen preparation should be filtered for 15 minutes, with more medium periodically being added to the SFC in order to maintain the medium at its original 3.0 ml. level. Occasionally during filtration, it may be necessary to aspirate media from the SFC using a pipette and then expirating it directly onto the top of the filter beads so as to prevent the build up of a film of dead sperm and debris which could block or interfere with proper flow of filtrate.

When filtration is complete, remove the SFC from the test tube and replace the bottom closure cap. Pull the filtrate from the test tube, centrifuge it, and reconstitute the spermatozoa at the desired level of dilution in a buffer of choice. The level of sperm dilution, or the sperm concentration in the resuspended preparation, shall be determined by the clinical purpose that the spermatozoa will be used for. For example, the filtered spermatozoa may be used for In Vitro Fertilization (IVF), Artificial Insemination (AI), Intrauterine Insemination (AIH-IU), Gamete Intrafollopian Transfer (GIFT) and other clinical techniques. Prior to actual use of the filtered-resuspended semen specimen, it should be assessed for sperm count per milliliter, motility, the presence of debris and other parameters routinely used in semenology. The filtered spermatozoa are now ready for use.

Table 1

Qualitative and quantitative characteristics of spermatozoa before and after filtration recovered through the Sephadex filtration process, as well as, recovered via the swim-up procedure using human spermatozoa

| | Qualitative - Quantitative Sperm Characteristics (N=12) | | | | | |
|---|---|---|---|---|---|---|
| | Motility (%) | Grade (0-4) | Morphology (% Normal) | Acrosomes (% Normal) | Debri Presence[2] (Subjective) | Sperm Recovered (x $10^6$/ml) |
| Before filtration | 57.6+5.1 | 3.1+0.6 | 61.3+7.0 | 57.4+7.1 | 3.0+0.9 | 78.3+9.1 |
| After filtration | 84.7+ | 3.7+0.4 | 87.4+4.1 | 88.3+5.1 | 0.8+0.1 | 31.2+5.3 |
| Swim-up[3] | 86.1+ | 3.7+0.5 | 82.4+5.2 | 81.0+6.2 | 0.7+0.1 | 17.1+4.4 |

[1] According to Zavos and Cohen, Fertil. Steril., 1980.

[2] Subjective evaluation; 0 = complete absence; 4 = severe. (Cohen et al., 1985).

[3] According to Cohen et al., Fertil. Steril., 1985.

## Claims

1. A semen filter device ccmprising a column having openings at both ends, dehydrated particulate filtering means in said column; said filtering means capable of being hydrated to form a multi-layer filter, and retaining means in said column to retain said dehydrated filtering means in said column.

2. The semen filter device of claim 1 including closure means for said openings and wherein said retaining means comprise a liquid pervious lower disc and an upper disc and said dehydrated filtering means are beads situated between said lower and top disc.

3. The semen filter device of claim 1 or 2 wherein the device is sterile and is enclosed in a wrapping that is sterile on the inside.

4. A sterile, disposable semen filtering device comprising a column having top and bottom openings, removable closure means for each of said top and bottom openings, retaining means in said column comprising a liquid-pervious lower disc and an upper disc, and dehydrated beads having a size from about 40 to 120 microns in diameter situated between said upper and lower disc; said beads capable of being hydrated to form a multi-layer filter.

5. The sterile, disposable semen filtering device of claim 1 enclosed in a wrapping that is sterile on the inside.

6. The method of filtering semen comprising hydrating the filtering means of a semen filter device of any one of claims 1 to 5 with a liquid media suitable for use with semen, for a time sufficient for the filtering means to hydrate, expand, and settle in said semen filter device to foam a multi-layer filter, adding to said multi-layer filter a washed semen specimen suspended in a liquid media suitable for use with semen, and recovering the semen that pass through the filter.

7. The method of claim 6 wherein the concentration of sperm in the liquid media prior to filtration is about $100 \times 10^6$ sperm/milliliter.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | BIOLOGICAL ABSTRACT Nb: 69034503 FAYEMI E.O. et al: " Assay of frozen boar semen with sephadex filtration." & THERIOGENOLOGY 12(1), 1979, p 13-18. | 1-7 | A61K35/52 A01K67/02 |
| Y | BIOLOGICAL ABSTRACT Nb: 84030191 OUM K.B. et al: " Separation of X and Y-bearing spermatozoa I. Separation of human spermatozoa by sephadex gel filtration." & KOREAN J.ANIM.SCI. 28(12), 1986, p 765-770. | 1-7 | |
| Y | MEDLINE ABSTRACT Nb: 78254914 SCHILLING E. et al: " Failure to separate human X- and Y-chromosome bearing spermatozoa by sephadex gel-filtration." & ANDROLOGIA, MAY-JUN 1978, 10(3), p 215-7. | 1-7 | |
| A | FR-A-2614899 (RANOUX C.J.E.) * the whole document * | 1-7 | |
| A | FR-A-2539628 (FONDATION DE RECHERCHE EN HORMONOLOGIE) * the whole document * | 1-7 | TECHNICAL FIELDS SEARCHED (Int. Cl.5 ) |
| A | WO-A-8702382 (PHARMACIA AB) * the whole document * | 1-7 | A61K A01K A61B |

The present search report has been drawn up for all claims

| Place of search | Date of completion of the search | Examiner |
|---|---|---|
| BERLIN | 20 AUGUST 1990 | AVEDIKIAN P.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document